(19) Europäisches Patentamt / European Patent Office / Office européen des brevets

(11) **EP 3 946 005 B1**

(12) **EUROPEAN PATENT SPECIFICATION**

(45) Date of publication and mention
of the grant of the patent:
**08.06.2022 Bulletin 2022/23**

(21) Application number: **20712368.8**

(22) Date of filing: **25.03.2020**

(51) International Patent Classification (IPC):
**A61B 5/00** (2006.01)    **A61K 8/00** (2006.01)
**A61Q 17/00** (2006.01)

(52) Cooperative Patent Classification (CPC):
**A61B 5/4848; A61B 5/445**

(86) International application number:
**PCT/EP2020/058246**

(87) International publication number:
**WO 2020/200936 (08.10.2020 Gazette 2020/41)**

(54) **POLLUTION PROTECTION FACTOR OF COSMETIC COMPOSITIONS**

VERSCHMUTZUNGSSCHUTZFAKTOR VON KOSMETISCHEN ZUSAMMENSETZUNGEN

FACTEUR DE PROTECTION CONTRE LA POLLUTION DE COMPOSITIONS COSMÉTIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.03.2019 PCT/CN2019/080317**
**07.05.2019 EP 19172948**

(43) Date of publication of application:
**09.02.2022 Bulletin 2022/06**

(73) Proprietors:
• **Unilever IP Holdings B.V.**
**3013 AL Rotterdam (NL)**
Designated Contracting States:
**AL AT BE BG CH CZ DK EE ES FI FR GR HR HU IS LI LT LU LV MC MK NL NO PL PT RO SE SI SK SM**
• **UNILEVER GLOBAL IP LIMITED**
**Wirral**
**Merseyside CH62 4ZD (GB)**
Designated Contracting States:
**CY DE GB IE IT MT RS TR**

(72) Inventors:
• **DOBKOWSKI, Brian, John**
**Trumbull, CT 06611 (US)**

• **HUANG, Nan**
**Shanghai, 201315 (CN)**
• **MENG, Sheng**
**Shanghai, 200335 (CN)**

(74) Representative: **James, Helen Sarah**
**Unilever PLC**
**Unilever Patent Group**
**Bronland 14**
**6708 WH Wageningen (NL)**

(56) References cited:
**CN-A- 106 290 176     CN-A- 108 785 201**

• **Michael Wong: "Review of PM2.5 and Air-Pollution Protection Factor (APPF) to Support Standardization of Testing Proposal", , July 2018 (2018-07), XP055639481, Calgary, Canada Retrieved from the Internet: URL:https://aquaskincare.ca/wp-content/upl oads/2018/07/Aqua-Skincare-APPF-white-pape r-2018.pdf [retrieved on 2019-11-06]**
• **DAVID MCDANIEL ET AL: "Atmospheric skin aging-Contributors and inhibitors", JOURNAL OF COSMETIC DERMATOLOGY, vol. 17, no. 2, 25 March 2018 (2018-03-25) , pages 124-137, XP055639595, GB ISSN: 1473-2130, DOI: 10.1111/jocd.12518**

EP 3 946 005 B1

## Description

### Field of the invention

The present invention relates to a method of evaluating the extent of protection offered by cosmetic compositions from atmospheric pollutants and using such evaluation to assess the efficacy of a composition and if necessary, compare it against other compositions.

### Background of the invention

The World Health Organization (WHO) reports that outdoor air pollution originates from natural and anthropogenic sources. While natural sources contribute substantially to local air pollution in arid regions more prone to forest fires and dust storms, there is substantial contribution from human activities.

Such human activities include fuel combustion, heat and power generation and industrial facilities (e.g. manufacturing factories, mines, and oil refineries). Pollutants could be categorized as particulate matter, black carbon, ground-level ozone and oxides of carbon, nitrogen and sulphur.

Particulate matter (PM) is inhalable particles composed of sulphate, nitrates, ammonia, sodium chloride, black carbon, mineral dust and water. Particles having a diameter of less than 10 microns ($PM_{10}$), including fine particles less than 2.5 microns ($PM_{2.5}$), pose maximum risks to health, as they can enter the lungs. Carbon black (soot) and dust (mineral oxides, such as iron oxides and the like) comprise much of the particulate matter in this range of particle size ranges.

Pollutants can cause several adverse effects on human skin, such as premature ageing, development of fine lines and wrinkles, pigmented spots, hyperpigmentation, rash and inflammation.

Some cosmetic compositions claim to prevent, inhibit or restrict the particulate pollutants from contacting our skin by forming a protective layer, i.e., they have ability to partly or fully block environmental pollutants like particles, oxide/superoxides and gases from contacting human skin. Formulation scientists often find it necessary to be able to substantiate such claims with evidence. Therefore, some manufacturing companies and some academic researchers have developed methods of testing/analysing or verifying the efficacy of such compositions. A purpose of such methods is to ascertain the efficacy of a candidate cosmetic composition. Sometimes the purpose is to compare the efficacy of one or more compositions or active ingredients, e.g. polymers. Some of these tests have been conducted on human volunteers. Some others have been conducted on suitable skin-equivalents such as plastic membranes, living-skin equivalents, Vitro-Skin®, *in vitro* skin models, *ex vivo* skin and the like. While human skin-equivalents is one component of such test methods, selection of an appropriate pollutant is equally important. However, it is not always possible to perform tests with actual pollutants, therefore model pollutants are often used.

Dow Corning has disclosed a test method to quantify the extent of protection conferred by its product, Splash Shield®, against particulate adhesion. A thin film of the test material is formed on collagen followed by surface analysis and exposure to carbon black repeat analysis.

EP1760440 A1 (P&G) discloses a method comprising the steps of:
selecting a Raman-active substance linked to the effectiveness of the skin care composition to be determined;

(i) measuring the concentration profile of said Raman-active substance as a function of depth within a test area of skin using Confocal Raman spectroscopy;
(ii) determining the thickness of the stratum corneum within said test area; then
(iii) applying the skin care composition to said test area; then
(iv) measuring the concentration profile of said Raman-active substance as a function of depth within said test area using Confocal Raman spectroscopy;
(v) determining the thickness of the stratum corneum within said test area; then
(vi) calculating the effectiveness of the skin care composition as a function of both; the concentration profile of the Raman-active substance after and before the application of the skin care composition; and the thickness of the stratum corneum after and before the application of the skin care composition.

Despite the information about a variety of test methods, there is no standard formula to assess the effects of anti-pollution cosmetic ingredients. The following webpage, *https://www.syntivia.fr/how-to-measure-anti-pollution-claims-in-vitro.html* discloses that there is no standard test or a calculation method to define a "Pollution Protection Factor" like SPF for sun protection compositions.

The webpage, "*https://www.cosmeticsdesign.com/Article/2017/10/05/Dow-invests-in-anti-pollution-skin-care-Part-2',* discloses that eventually a Pollution Protection Factor could be developed by the industry.

The webpage, *https://www.marieclaire.co.uk/beauty/skincare/the-10-best-anti-pollution-products-for-skin-*

*295167,* also discloses that at present there is no standardized term called Pollution Protection Factor.

KR100373519B (Pacific Corporation, 2003) discloses a skin pollution index corresponding to climatic factors considering influence of climatic factors make the best use of the skin pollution index for skin care. The skin pollution index is formed with climatic factors influencing skin contamination as humidity, average temperature, wind velocity and air pollution warning. The climatic factors are graded according to influence on pollution of skin, and each grade is digitized. The skin pollution index is set with operating values according to grades about humidity, average temperature, wind velocity and air pollution warning. Ten grades are set, and the weight of twenty is added in alarming the air pollution warning. The skin pollution index per day is displayed numerically corresponding to climatic factors per day, and information on skin care is provided efficiently with alarming the present state of skin pollution according to the skin pollution index.

However, this factor is for weather forecasting rather than for cosmetic compositions.

US2008187502 A1 (Michelle Garay) discloses that conventional methods for assessing the effects of oxidative stress on skin involve invasive methods for harvesting skin cells (such as via skin biopsy), costly clinical studies, or invasive methods of collecting viable cells. Conventional in vitro methods attempt to assess oxidative stress by simulating the effects of external aggressions on "cultured" cells obtained in various manners, and thus measure effects that do not capture a complete biological response. Therefore, the inventors therein have disclosed a method of assessing oxidative stress in a mammal which comprises the steps of exposing skin cells of said mammal to an oxidizable moiety, exposing said skin cells to an external aggression, and assessing a reaction product of said oxidizable moiety. Prior to the assessing step, said skin cells are non-invasively removed from said mammal such that said removed skin cells are viable. Using this method, the Oxidation Protection Factor, OPF, of an extrinsic composition is measured and evaluated. The OPF of the extrinsic composition, X, is determined using the method of the invention and the following equation:

$$OPF = 100 * (S_{N,\,UV} - S_{X,\,UV})/(S_{N,\,UV} - S_{N,\,NUV})$$

where SN,UV is the fluorescence signal obtained when no extrinsic composition is applied to the skin cells, which are then exposed to UV radiation;

where SN,NUV is the fluorescence signal obtained when no extrinsic composition is applied to the skin cells, which are not exposed to UV; and

where SX,UV is the fluorescence signal obtained when extrinsic composition X is applied to the skin cells, which are then exposed to UV radiation.

HERRLING, T. The Radical Status Factor (RSF): a novel metric to characterize skin products. Int J Cosmet Sci. August 2012, Vol 34(4), pages 285-90. discloses a Radical Status Factor (RSF). The RSF describes the properties of a substance to protect against or to promote generation of free radicals. The existence of free radicals is strongly correlated to all redox processes that take place in the skin. So is in the case of sun exposure. Substances that have a good protection against free radicals are marked by factors which are RSF = 1. UV filters have the highest RSF. Radical protection of RSF = 100 is the currently upper limit of radical protection as it allows only 1% radical generation. A moderate protection is reached by using antioxidants. They can act as a second defense line against sun damage. RSF > 2, meaning 50% radical protection, is said to be hard to achieve. But antioxidants can penetrate in the dermis to protect the skin from the inner side contrary to UV filter, which are deposited on the surface only. The normal untreated skin is characterized by RSF = 1, which means no protection against and no promotion of free radicals. Such information could be useful to formulate more efficacious compositions, or to be able to choose one composition over few others in view of their efficacy.

The factor disclosed in this publication is a metric for grading sun screen products.

CN08785201A discloses a cosmetic product containing an antioxidant and a barrier enhancer, wherein its abilities to inactivate a pollutant and to inhibit the pollutant from contacting the skin are tested.

CN106290176A discloses testing the ability of a cosmetic product to remove a pollutant from the skin.

Dermatec Lyon Company has disclosed a method for determining pollution protection factor of a cosmetic composition which is done in a 2-step process. In the first step it is necessary to compare the effect of exposure of skin to cigarette smoke for a period of 15 minutes. Thereafter, treated and untreated areas of the skin are compared to assess the antioxidant effect of a cosmetic composition. By the procedure disclosed in this publication, the cosmetic composition is found to have a pollution protection factor (PPF) of 20.

An unmet need exists for a robust and reliable method to determine the pollution protection factor of any cosmetic composition and such a PPF could be used as a tool to assess/grade cosmetic compositions based on their efficacy as far as protection from the harmful effects of pollution is concerned. The present invention addresses the needs by overcoming at least one drawback, disadvantage or limitation of the state of the art.

## Summary of the invention

<u>The Factor</u>

Consumers expect that an efficacious cosmetic composition should render ineffective at least one type of pollutant present on the skin, or it should prevent contact of at least one type of pollutant with the skin or it should remove (cleanse) at least one type of pollutant present on the skin. Accordingly, the pollution protection factor of a cosmetic composition can be seen as an indicator of its ability to protect the skin by at least one of the aforementioned modes of action.

In accordance with the invention a method of determination of a pollution protection factor (PPF) of a cosmetic composition is provided, as defined by claim 1.

## Detailed description of the invention

In accordance with the present invention, the extent of protection offered by a cosmetic composition against atmospheric pollutants is represented or expressed as a factor called (Pollution Protection Factor, PPF).

It is preferred that factor (A, B or C as above) i.e., said PPF, is a number between 0 and 75, where 0 represents the least or no protection while 75 represents the maximum possible protection that can be offered by a cosmetic composition. The numerical range (or spread) of 0 to 75 is realistic and would seem familiar to consumers because they know of the sun protection factor (SPF) as applicable to cosmetic compositions which is also a number in about the same range.

In each of the equations A to F. The denominator 0.047 is a constant and its use ensures that PPF is in the range of 0 to 75.

It is preferred that the PPF of a cosmetic composition so determined is associated with the cosmetic composition, for example with a packaging of the composition or in an audio-visual communication concerning the composition. Preferably the PPF of the cosmetic composition is associated with a packaging of the cosmetic composition. It is preferred that the packaging is a primary packaging. Preferably the primary packaging is a tube, sachet, pouch, bottle or a dispenser. Alternatively, it is preferred that the packaging is a secondary packaging, preferably a carton or a box. By associated with the packaging' means that the packaging bears or carries information about the PPF of the concerned cosmetic composition which is in the form of the PPF (preferably 0 to 75) or in the form of at least one indicium as described hereinafter.

Once a PPF of a cosmetic composition has been assigned or determined by the method in accordance with the invention, the concerned number on the scale of 0 to 75 is preferably further represented in at least one secondary non-numerical form. It is preferred that such a non-numerical form includes tactile, visual or olfactory form. It is preferred that such visual form is in the form of an indicium such an alphabet or a set of words such as high/medium/low or good/better/best or a colour scale or shade card or a colour-based indicium like red/amber/green where red represents a lower factor, e.g. less than 10 and green colour represents a higher factor such as 60 or 65.

For example, it is preferred that when the PPF is in the range of 60 to 75, it is termed as high; when it is in the range of 45 to 59 it is termed medium and when it is in the range of 10 to 44 it is termed low.

Alternatively, the factor is represented in the form of smiley faces or emoticons which are well known in social media. When the indicium is tactile it preferably is the form of indentations or raised projections bearing a sign or symbol sufficiently indicative of the PPF or a certain number of projections that could be easily read and understood by a person who understands such signs. The pollution protection factor could be secondarily represented in a variety of ways and the aforementioned examples are non-limiting.

The present invention relates to a scalable and consumer-friendly method to assess the antipollution efficacy of a cosmetic compositions including but not limited to skin care, skin cleansing and hair care products. Such a method could be used to assess the efficacy of any composition, regardless of whether such composition claims to protect from pollution or otherwise. Such compositions usually contain one or more ingredients that individually or collectively offer certain extent of protection from pollution. This scalable factor (PPF) is a usable and reliable guide for selection of cosmetic compositions that protect our skin from the ill effects of atmospheric pollutants. In addition, this factor could also be used in any form of communication of the efficacy or benefits of such cosmetic compositions via media such as print, TV or social media in the form of e.g., advertisement. Further, such PPF could be used to substantiate any claims, such as consumer claims or advertisement claims pertaining to the benefits or efficacy of cosmetic compositions. Alternatively, the method according to the invention is useful for comparing the pollution protection factor (efficacy) of a given cosmetic composition with one or more other cosmetic compositions.

The present invention is based, at least partly, on the assumption that any cosmetic composition which can protect the skin of a user from harmful effects of atmospheric pollution comprises at least one ingredient (active) which either renders ineffective at least one type of pollutant present on the skin (i.e., neutralises) or which inhibits contact of at least one type of pollutant with skin (i.e. blocks) or which removes at least one type of pollutant present on the skin (i.e. removes).

The Efficacy α

This step is a step of testing, by any known test method, the ability (α) of the cosmetic composition to render ineffective at least one type of pollutant present on the skin and expressing it on a scale of 0 to 1, followed by calculating the PPF by equation A, where (1 - α) indicates corresponding potential residual damage to the skin.

Potential residual damage represents the extent of damage that can be caused by the pollutant as determined by the concerned assay.

The composition comprises at least one ingredient known to render ineffective said at least one type of pollutant. In other words, in the method of the invention, the efficacy (α) is attributed to presence of at least one ingredient in said composition known to render ineffective said at least one type of pollutant.

The at least one ingredient is an anti-inflammatory or an antioxidant.

It is preferred that when the ingredient is found to have a comparatively weaker anti-inflammatory effect said ability (α) is taken to be 0.2 and when said ingredient is found to have a comparatively stronger anti-inflammatory effect said ability (α) is taken to be 0.75, and correspondingly said residual damage is 0.8 and 0.25. For example, niacinamide has weak(er) anti-inflammatory effect in which case the ability (α) is taken to be 0.25. It is preferred that the anti-inflammatory ability is tested by cell-based cytokine assay. It is preferred that the antioxidant effect is tested by cellular ROS assay. However, the anti-inflammatory ability could alternatively be determined by any other suitable method.

On the other hand, when 12-HSA is present in the cosmetic composition the value of efficacy (α) is taken to be 0.5 and when the composition comprises curcumin the value of efficacy (α) is taken to be 0.7 because curcumin is highly potent anti-inflammatory agent. Further, when the ingredient has comparatively weaker antioxidant effect said ability (α) is taken to be 0.2 and when the ingredient has comparatively stronger antioxidant effect said ability (α) is taken to be 0.8, and correspondingly the potential residual damage is in the range of 0.8 to 0.2. An example of a comparatively weaker antioxidant is resorcinol, an example of an ingredient having comparatively stronger antioxidant effect is flavonoid and glutathione (GSH). An ingredient having antioxidant effect midway is vitamin C.

Preferably the antioxidant is vitamin C or a derivative, e.g., ascorbyl glucoside, phenol, polyphenols such as tannins, ellagic acid and tannic acid, tea extracts such as green tea extracts; anthocyanins; rosemary extracts; phenol acids, stilbenes in particular resveratrol, derivatives of sulphur amino acids such as S-carboxymethyl-cysteine; ergothioneine; N-acetylcysteine, carotenoids, retinoic acid, retinol, flavonoids, vitamin E, sulfated polysaccharides or a lignan.

It is preferred that the step of testing, by any known test method, the ability (α) of the cosmetic composition to render ineffective at least one type of pollutant present on the skin is carried out on a 3D living skin-equivalent (LSE) which is also known as human skin-equivalent (HSE), i.e. a material that resembles the skin of human beings. The living skin-equivalent comprises viable keratinocytes. It also comprises a barrier layer differentiated into stratum corneum. Currently, several LSE are commercially available for such applications. Suitable examples include EpiKutis®, MelaKutis®, EpiSkin ®, SkinEthic RHE®, SkinEthic RHPE®, EpiDerm®, T-skin®, MelanoDerm®, EpiDermFT®. These HSEs could be divided into two major categories: epidermal and full-thickness models.

The Efficacy β

Furthermore, the method in accordance with the invention comprises a step of testing, by any known test method, the ability (β) of the cosmetic composition to inhibit the contact of at least one type of pollutant with skin and expressing it also on a scale of 0 to 1, where (1 - β) indicates corresponding potential residual damage to the skin. The composition comprises at least one ingredient known to inhibit the contact of at least one type of pollutant with skin. In other words, the efficacy (β) is attributed to the presence of at least one ingredient in the composition known to prevent contact of at least one type of pollutant with skin. This at least one ingredient is a sunscreen, a barrier enhancer or a polymer.

Further preferably if the SPF (sun protection factor) of said sunscreen is greater than 15, the corresponding ability (β) is taken to be 0.5 and if SPF of said sunscreen is less than or equal to 15, the corresponding ability (β) is taken to be 0.2 and correspondingly the potential residual damage is 0.5 and 0.8.

Preferably the SPF is determined by an ASTM method.

Alternatively, when a strong barrier enhancer is present the corresponding ability (β) is taken to be 0.7 and where a weak barrier enhancer is present, the corresponding ability (β) is taken to be 0.2 and correspondingly the potential residual damage is 0.3 and 0.8.

Further, when, in view of the presence of the polymer, the inhibition of contact of said at least one type of pollutant with skin is found to be >70%, the corresponding ability (β) is assigned a score of 0.7 on the scale of 0 to 1 and when said inhibition is found to be <30% the corresponding ability (β) is assigned a score of 0.2 on the scale of 0 to 1 and correspondingly the potential residual damage is 0.3 and 0.8. The % blocking can be determined by any suitable method known in the art.

For example, when % blocking in view of the presence of e.g. herbal extracts, is <30%, the corresponding efficacy ($\beta$) is taken to be 0.2. An example of a suitable ingredient is certain plant extracts containing proteins such as Purisoft®. When the % blocking in view of the presence of e.g., a cumulative deposition polymer is in the intermediate range (30 to 70% blocking), the corresponding efficacy is taken to be 0.5. Certain cumulative deposition polymers fall in this class.

When the barrier enhancement is stronger the corresponding efficacy ($\beta$) is taken to be 0.7. An example of a suitable ingredient is a PPAR activator like 12-HSA. When the barrier enhancement is of intermediate range the corresponding efficacy is taken to be 0.5. An example of a suitable ingredient is glycerol. When the barrier enhancement is low, the corresponding efficacy is taken to be 0.3. An example of an ingredient that brings about low barrier enhancement is sunflower seed oil.

It is preferred that $\beta$ is measured by pollutant penetration assay or by ex-vivo barrier model. A variety of such methods is freely available on the internet and in journals, books and periodicals.

The Efficacy (v)

The composition comprises at least one ingredient known to remove at least one type of pollutant present on the skin. In other words, in the method in accordance with the invention the efficacy ($\gamma$) is attributed to the presence of at least one ingredient in the composition known to remove at least one type of pollutant present on the skin. In such a case, when % removal (of the pollutant) in view of the ingredient is 95 to 100%, the corresponding efficacy ($\gamma$) is taken to be 0.8 and when the % removal in view of said ingredient is 75 to 85%, the corresponding efficacy ($\gamma$) is taken to be 0.4, and correspondingly the potential residual damage is 0.2 and 0.6. In other words, it is preferred that the said composition comprises at least one ingredient known to remove at least one type of pollutant present on the skin.

Preferably when said composition is a cleanser which removes 75 to 85% of said at least one type of pollutant present on the skin the corresponding ability is assigned a score of 0.4 on the scale of 0 to 1 and when said composition is a cleanser which removes 85 to 95% of said at least one type of pollutant present on the skin the corresponding ability is assigned a score of 0.7 on the scale of 0 to 1, and when said composition is a cleanser which removes 95 to 100% of said at least one type of pollutant present on the skin the corresponding ability is assigned a score of 0.8 on the scale of 0 to 1, and correspondingly the potential residual damage is 0.6, 0.3 and to 0.2.

In accordance with the invention, the method comprises, in no particular order:

(a) a step of testing, by any known test method, the ability ($\alpha$) of the cosmetic composition to render ineffective at least one type of pollutant present on the skin and expressing it on a scale of 0 to 1, where ($1 - \alpha$) indicates corresponding potential residual damage to the skin;
(b) a step of testing, by any known test method, the ability ($\beta$) of the cosmetic composition to inhibit the contact of at least one type of pollutant with skin and expressing it also on a scale of 0 to 1, where ($1 - \beta$) indicates corresponding potential residual damage to the skin, further followed by;
(c) a step of testing, by any known test method, the ability ($\gamma$) of the cosmetic composition to remove at least one type of pollutant present on the skin, expressing it also on a scale of 0 to 1, where ($1 - \gamma$) indicates corresponding potential residual damage to the skin and calculating the pollution protection factor by the equation F.

| F |
|---|
| $$PPF = -\frac{\log{(1-\alpha)} \times (1-\beta) \times (1-\gamma)}{0.047}$$ |

Therefore, according to the invention, the method has one step in which the ability ($\alpha$) of the cosmetic composition to render ineffective at least one type of pollutant present on the skin is determined.

This is followed by a second step of testing the ability ($\beta$) of the cosmetic composition to prevent contact of at least one type of pollutant with skin and expressing it also on a scale of 0 to 1.

This is further followed by a third step of testing the ability ($\gamma$) of the cosmetic composition to remove at least one type of pollutant present on the skin and expressing it also on a scale of 0 to 1.

The factor represents cumulative ability of rendering ineffective a pollutant and preventing contact of at least one type of pollutant with skin and remove at least one type of pollutant present on the skin. The factor is expressed as a sum-total of the ability to neutralise, block and remove such pollutants, if the concerned cosmetic composition can perform all three functions. The term "sum-total" does not mean mathematical addition of the three individual effects but rather it means a holistic index arrived at or derived by performing an algorithm, where each effect contributes to the holistic index or factor (PPF) albeit to different extent depending on the nature and purpose of the concerned composition and depending on the efficacy of each ingredient concerned. For example, a leave-on skin care cream contributes very little towards removal of pollutants but could play a significant role in blocking or neutralizing (i.e. rendering ineffective) the ill effects of atmospheric pollutants. On the other hand, a wash-off product, such as a facewash, would primarily remove pollutants and it would be anticipated that there would be very little contribution towards neutralisation and preventing contact.

As used herein the term "comprising" encompasses the terms "consisting essentially of" and "consisting of". Where the term "comprising" is used, the listed steps or options need not be exhaustive. Unless otherwise specified, numerical ranges expressed in the format "from x to y" are understood to include x and y. In specifying any range of values or amounts, any particular upper value or amount can be associated with any particular lower value or amount. As used herein, the indefinite article "a" or "an" and its corresponding definite article "the" means at least one, or one or more, unless specified otherwise. The various features of the present invention referred to in individual sections above apply, as appropriate, to other sections *mutatis mutandis.* Consequently, features specified in one section may be combined with features specified in other sections as appropriate. Any section headings are added for convenience only, and are not intended to limit the disclosure in any way. The examples are intended to illustrate the invention and are not intended to limit the invention to those examples *per se.*

The term cosmetic composition means any cosmetic composition. Preferably the cosmetic composition is a leave-on cosmetic composition. Alternatively, is a wash off composition. "Leave-on" as used herein means compositions that are applied to the skin and are not intended to be washed or rinsed off for some time, as contrasted with cleansing or wash-off or rinse-off compositions.

Preferably the leave-on cosmetic composition is a serum, hand creams, face creams, body lotions, make up compositions such as foundations, lipsticks, hair styling gels, hair styling creams and deodorants and antiperspirants such as roll on or sticks. The compositions may accordingly be in a variety of formats.

The term active ingredient means any ingredient, including film forming polymers present in the cosmetic composition to inhibit (or neutralize or wash) the contact of particulate pollutants with human skin. Non-limiting examples thereof include silicone polymers and extracts of natural products such as extract of roots or leaves of any plant.

Human skin acts like a natural shield which protects our body from external influences. However, at times, and under certain conditions, the skin may no longer perform this function fully and efficiently. There is plethora of literature to substantiate the statement that atmospheric pollutants affect the normal functioning of human skin. Particulate pollutants tend to top the list at least in some countries or some regions of the world.

Often formulation scientists have explored newer and more effective cosmetic compositions to protect our skin from pollutants, including the compositions or active agents which can resist, restrict or prevent the contact of such pollutants with skin. However, as discussed at length under the section of background and prior art, there is need for a more robust and reliable method for assessment of efficacy of such compositions. The present invention addresses such as need, at least in part.

Without wishing to be bound by theory it is believed that the principle of assessment of pollution protection factor is to correlate a kind of protection (from pollution) by way of an individual ingredient or by way of ingredients having same or similar mechanism of action (MoA). Eventually the respective benefits are combined as it is presumed that when present, each ingredient would perform its defined role on the skin or the user. The net effect is presented as a single holistic factor or an index that can be used to show or demonstrate the efficacy of the composition in total.

Table 1 contains information about the three main mechanisms of action which could be linked or correlated with some of the important functional ingredients usually included in cosmetic compositions.

**Table 1**

| Action | Exemplary modes of action |
|---|---|
| The ability ($\alpha$) of the cosmetic composition to render ineffective at least one type of pollutant present on the skin | a. Reduction of oxidative damage |
| | b. Inhibition of inflammatory signaling |
| The ability ($\beta$) of the cosmetic composition to inhibit the contact of at least one type of pollutant with skin | a. Repelling/trapping/isolating pollutants above the surface of the skin and inhibiting their passage through the layer of the cosmetic composition |
| | b. Blocking UV energy and prevent photo-pollution damage |
| | c. Strengthen or maintain healthy skin's natural barrier |

(continued)

| Action | Exemplary modes of action |
|---|---|
| The ability ($\gamma$) of the cosmetic composition to remove at least one type of pollutant present on the skin | a. The concerned cosmetic composition or ingredient washes away the pollutant |

Depending on the formulation of the concerned cosmetic composition, the functionally active ingredients of the composition are identified.

Then depending on the nature of the functional activity of the active ingredient, a sub-score from 0 to 1 is allotted to the composition for its ability ($\alpha$, $\beta$ or $\gamma$ as the case may be). This sub-score corresponds to the performance of the concerned ingredient that is known to deliver a benefit through a specific mode of action. For example, a product containing safe and effective amount of vitamin C is considered to have medium level of anti-oxidant efficacy and therefore it is allotted a sub-score of 0.5. Each sub-score is in the range of 0 to 1. A score of 1 means that the ingredient provides maximum possible protection through a mode of action. Correspondingly, and at least theoretically, it means that the ingredient prevents any damage to the skin. On the contrary, a score of 0 means that theoretically there is no protection as far as this mode of action is concerned. Thus, the higher the score, the better the efficacy. Scientifically proven in vitro or in vivo tests are available for each mode of action which could be used to determine the extent of protection.

When the individual scores are multiplied, it provides an indication of the cumulative damage (or the extent of non-protection) In view of the use of negative logarithm, the holistic efficacy is inversely proportional to the cumulative damage; it is higher if the cumulative damage is lower.

The equation followed in the method of the invention can be used to ascertain holistic efficacy of any cosmetic composition and the effect of addition or removal of any ingredient in the composition on the holistic efficacy can be easily determined by adding or subtracting the efficacy corresponding to the concerned ingredient from the holistic efficacy.

Therefore, the present invention provides a method by which holistic efficacy of any cosmetic composition of a known or unknown formulation or even the holistic efficacy achieved through a cosmetic regimen, the method in accordance with the invention is robust enough to permit analysis, without requiring repetitive confirmatory tests. At the same time, there is little or no possibility of conflicting results between products containing the same ingredient.

The term particulate pollutant, also called particulate matter or PM, means a mixture of solids and liquid droplets floating in the air. Some particles are released directly from a specific source, while others form in complicated chemical reactions in the atmosphere. Suitable examples include dust, dirt, soot, or smoke. Particulate pollutants are described in terms of particle size: $PM_{2.5}$ and $PM_{10}$ having an aerodynamic diameter less than 2.5 $\mu$m and 10 $\mu$m, respectively. It is preferred that in the method in accordance with the invention, the model fine particulate matter resembles $PM_{2.5}$ or $PM_{10}$ at least in size.

The cosmetic composition

The cosmetic composition whose pollution protection factor is to be determined, is preferably the one that can be directly applied to the skin. Alternatively, they can be delivered by various transdermal delivery systems, such as transdermal patches as known in the art. For example, the cosmetic composition could be a solution, gel, lotion, ointment, cream, suspension, paste, liniment, powder, tincture, aerosol, patch, or the like, in a cosmetically acceptable base.

The composition may further be in a variety of product types that include but are not limited to toners, sticks, sprays, ointments, pastes, foams, powders, mousses, strips, patches, electrically-powered patches, hydrogels, film-forming products, facial and skin masks, make-up such as foundations and the like.

The invention will now be described in detail with the following non-limiting examples.

**Examples**

A few cosmetic compositions of known formulations were chosen for the experiments. They were a cleanser, a day cream, a night cream, body lotion and a light hydration gel. Actives commonly used in such/or give detailed formulations.

The compositions were subjected to a series of analysis which are as follows:

-    $\alpha$ was determined by the using fluorescent particles on Vitro Skin™

-    $\beta$ was determined by pollutant penetration assay, alternatively by ex-vivo barrier model

- γ was determined by cellular ROS assay or cell-based cytokine assay or by measurement of SPF

The observations are summarised in Table 2.

**Table 2**

| Details of Testing Method | Skin Cleanser | Day Cream | Night Cream | Body Lotion | Light Hydration Gel |
|---|---|---|---|---|---|
| **Fluorescent Particles on Vitro Skin™** | | | | | |
| Score/Ability ($\alpha$) | 0.80 | 0.00 | 0.00 | 0.00 | 0.00 |
| Potential Residual Damage | 0.20 | 1.00 | 1.00 | 1.00 | 1.00 |
| **Pollutant Penetration Assay** | | | | | |
| Score/Ability ($\beta$) | 0.00 | 0.80 | 0.00 | 0.50 | 0.00 |
| Potential Residual Damage | 1.00 | 0.20 | 1.00 | 0.50 | 1.00 |
| **Ex vivo Barrier Model** | | | | | |
| Score/Ability | 0.50 | 0.70 | 0.30 | 0.70 | 0.50 |
| Potential Residual Damage | 0.50 | 0.30 | 0.70 | 0.30 | 0.50 |
| **Cellular ROS assay** | | | | | |
| Score/Ability ($\gamma$) | 0.00 | 0.80 | 0.80 | 0.20 | 0.50 |
| Potential Residual Damage | 1.00 | 0.20 | 0.20 | 0.80 | 0.50 |
| **Cell-based Cytokine Assay** | | | | | |
| Score/Ability ($\gamma$) | 0.00 | 0.50 | 0.75 | 0.25 | 0.25 |
| Potential Residual Damage | 1.00 | 0.50 | 0.25 | 0.75 | 0.75 |
| **SPF** | | | | | |
| Score/Ability ($\gamma$) | 0.00 | 0.50 | 0.00 | 0.00 | 0.00 |
| Potential Residual Damage | 1.00 | 0.50 | 1.00 | 1.00 | 1.00 |
| **Total Potential Residual Damage** | 0.50 | 0.00 | 0.04 | 0.09 | 0.19 |
| PPF** | 21 | 54 | 31 | 22 | 15 |
| ** As calculated according to equation F | | | | | |

The illustrated examples clearly indicate that the method in accordance with the invention is a robust and reliable tool for measuring and comparing efficacies of cosmetic compositions against atmospheric pollutants. The method could be used to measure and demonstrate cleansing efficacy of cosmetic cleansing compositions against atmospheric pollutants, especially particulate pollutants such as $PM_{2.5}$ and $PM_{10}$ or oxidative pollutants like ozone and peroxides. Outcome of the method could potentially be useful for relative ranking of various cosmetic compositions belonging to the same category of products, such as a soap-based cleanser v/s a non-soap surfactant-based cleanser, or even, where necessary, between two or more products belonging to different categories of products for example, a face cream against a body lotion. The method could also be useful to determine the efficacy of one or more active ingredients such as film-forming agents and polymers by suitably formulating the candidate compositions to be tested. Further, the method of the invention could also be used as a demonstration tool for consumer promotion or activation of new or existing cosmetic cleansing compositions. Outcome of the method could also be used for claim-support. Further, the holistic efficacy of a cosmetic composition so assessed could be used in communications or even be printed as back-of-pack information.

**Claims**

1. A method of determination of a pollution protection factor (PPF) of a cosmetic composition, comprising:

    (a) a step of testing, by any known test method, the ability ($\alpha$) of the cosmetic composition to render ineffective at least one type of pollutant present on the skin and expressing it on a scale of 0 to 1, where (1 - $\alpha$) indicates corresponding potential residual damage to the skin, wherein said composition comprises at least one ingredient known to render ineffective said at least one type of pollutant and said at least one ingredient is an anti-inflammatory or antioxidant;
    (b) a step of testing, by any known test method, the ability ($\beta$) of the cosmetic composition to inhibit the contact of at least one type of pollutant with skin and expressing it also on a scale of 0 to 1, where (1 - $\beta$) indicates corresponding potential residual damage to the skin wherein said composition comprises at least one ingredient known to inhibit the contact of at least one type of pollutant with skin and wherein said ingredient is a sunscreen, a barrier enhancer or a polymer, further followed by;
    (c) a step of testing, by any known test method, the ability ($\gamma$) of the cosmetic composition to remove at least one type of pollutant present on the skin, expressing it also on a scale of 0 to 1, where (1 - $\gamma$) indicates corresponding potential residual damage to the skin, and calculating the pollution protection factor by the equation F.

| F |
|---|
| $$PPF = -\frac{\log (1 - \alpha) \times (1 - \beta) \times (1 - \gamma)}{0.047}$$ |

2. A method as claimed in claim 1 wherein when said ingredient is found to have a comparatively weaker anti-inflammatory effect said ability ($\alpha$) is taken to be 0.2 and when said ingredient is found to have a comparatively stronger anti-inflammatory effect said ability ($\alpha$) is taken to be 0.75, and correspondingly said potential residual damage is 0.8 and 0.25.

3. A method as claimed in claim 1 or 2 wherein when said ingredient is found to have a comparatively weaker antioxidant effect said ability ($\alpha$) is taken to be 0.2 and when ingredient is found to have a comparatively stronger anti-inflammatory effect said ability ($\alpha$) is taken to be 0.8, and correspondingly the potential residual damage is 0.8 and 0.2.

4. A method as claimed in any of the preceding claims wherein if the SPF (sun protection factor) of said sunscreen is greater than 15, the corresponding ability ($\beta$) is taken to be 0.5 and if SPF of said sunscreen is less than or equal to 15, the corresponding ability ($\beta$) is taken to be 0.2 and correspondingly the potential residual damage is 0.5 and 0.8.

5. A method as claimed in any of the preceding claims wherein when a strong barrier enhancer is present the corresponding ability ($\beta$) is taken to be 0.7 and where a weak barrier enhancer is present, the corresponding ability ($\beta$) is taken to be 0.2 and correspondingly the residual damage is in the range of 0.3 to 0.8.

6. A method as claimed in any of the preceding claims wherein when, in view of the presence of the polymer, the inhibition of the contact of said at least one type of pollutant with skin is found to be >70%, the corresponding ability ($\beta$) is assigned a score of 0.7 on the scale of 0 to 1 and when said inhibition is found to be <30% the corresponding ability ($\beta$) is assigned a score of 0.2 on the scale of 0 to 1 and correspondingly the residual damage is 0.3 and 0.8.

7. A method as claimed in any of the preceding claims wherein said composition comprises at least one ingredient known to remove at least one type of pollutant present on the skin.

8. A method as claimed in any of the preceding claims wherein when said composition is a cleanser which removes 75 to 85% of said at least one type of pollutant present on the skin the corresponding ability is assigned a score of 0.4 on the scale of 0 to 1 and when said composition is a cleanser which removes 85 to 95% of said at least one type of pollutant present on the skin the corresponding ability is assigned a score of 0.7 on the scale of 0 to 1, and when said composition is a cleanser which removes 95 to 100% of said at least one type of pollutant present on the skin the corresponding ability is assigned a score of 0.8 on the scale of 0 to 1, and correspondingly the residual damage is 0.6, 0.3 and to 0.2.

9. A method as claimed in any of the preceding claims wherein said PPF is a number between 0 and 75.


**Patentansprüche**

1. Verfahren zum Bestimmen eines Schadstoffschutzfaktors (PPF) einer kosmetischen Zusammensetzung, wobei das Verfahren die folgenden Schritte umfasst:

   (a) einen Schritt zum Testen durch ein bekanntes Testverfahren der Fähigkeit ($\alpha$) der kosmetischen Zusammensetzung, wenigstens einen Typ Schadstoff, der auf der Haut vorhanden ist, unwirksam zu machen, und diese auf einer Skala von 0 bis 1 auszudrücken, wobei ($1 - \alpha$) entsprechend eine mögliche verbleibende Schädigung der Haut angibt, wobei die Zusammensetzung wenigstens einen Inhaltsstoff umfasst, der dafür bekannt ist, den wenigstens einen Typ Schadstoff unwirksam zu machen, und wobei der wenigstens eine Inhaltsstoff ein entzündungshemmendes Mittel oder ein Antioxidationsmittel ist;
   (b) einen Schritt zum Testen durch ein bekanntes Testverfahren der Fähigkeit ($\beta$) der kosmetischen Zusammensetzung, den Kontakt von wenigstens einem Typ Schadstoff mit Haut zu verhindern, und diese ebenfalls auf einer Skala von 0 bis 1 auszudrücken, wobei ($1 - \beta$) entsprechend eine mögliche verbleibende Schädigung der Haut angibt, wobei die Zusammensetzung wenigstens einen Inhaltsstoff umfasst, der dafür bekannt ist, den Kontakt von wenigstens einem Typ Schadstoff mit Haut zu verhindern, und wobei der Inhaltsstoff ein Sonnenschutz, ein Barriereverstärker oder ein Polymer ist, ferner gefolgt von:
   (c) einem Schritt zum Testen durch ein bekanntes Testverfahren der Fähigkeit ($\gamma$) der kosmetischen Zusammensetzung, wenigstens einen Typ Schadstoff, der auf der Haut vorhanden ist, zu entfernen, wobei diese ebenfalls auf einer Skala von 0 bis 1 ausgedrückt wird, wobei ($1 - \gamma$) eine entsprechende mögliche verbleibende Schädigung der Haut angibt, und zum Berechnen des Schadstoffschutzfaktors durch die Gleichung F.

$$F: \qquad PPF = \frac{-\log(1 - \alpha) \times (1 - \beta) \times (1 - \gamma)}{0{,}047}.$$

2. Verfahren nach Anspruch 1, wobei dann, wenn herausgefunden wird, dass der Inhaltsstoff eine vergleichsweise schwächere entzündungshemmende Wirkung hat, die Fähigkeit ($\alpha$) als 0,2 angenommen wird, und wenn herausgefunden wird, dass der Inhaltsstoff eine vergleichsweise stärkere entzündungshemmende Wirkung hat, die Fähigkeit ($\alpha$) als 0,75 angenommen wird, und wobei entsprechend die mögliche verbleibende Schädigung 0,8 und 0,25 beträgt.

3. Verfahren nach Anspruch 1 oder 2, wobei dann, wenn herausgefunden wird, dass der Inhaltsstoff eine vergleichsweise schwächere Antioxidationswirkung hat, die Fähigkeit ($\alpha$) als 0,2 angenommen wird, und wenn herausgefunden wird, dass der Inhaltsstoff eine vergleichsweise stärkere entzündungshemmende Wirkung hat, die Fähigkeit ($\alpha$) als 0,8 angenommen wird, und wobei entsprechend die mögliche verbleibende Schädigung 0,8 und 0,2 beträgt.

4. Verfahren nach einem der vorhergehenden Ansprüche, wobei dann, falls der SPF (Sonnenschutzfaktor) des Sonnenschutzes größer als 15 ist, die entsprechende Fähigkeit ($\beta$) als 0,5 angenommen wird, und falls der SPF des Sonnenschutzes kleiner oder gleich 15 ist, die entsprechende Fähigkeit ($\beta$) als 0,2 angenommen wird, und wobei entsprechend die mögliche verbleibende Schädigung 0,5 und 0,8 beträgt.

5. Verfahren nach einem der vorhergehenden Ansprüche, wobei dann, wenn eine starke Barriereverstärkung vorhanden ist, die entsprechende Fähigkeit ($\beta$) als 0,7 angenommen wird, und wenn eine schwache Barriereverstärkung vorhanden ist, die entsprechende Fähigkeit ($\beta$) als 0,2 angenommen wird, und wobei entsprechend die verbleibende Schädigung im Bereich von 0,3 bis 0,8 liegt.

6. Verfahren nach einem der vorhergehenden Ansprüche, wobei dann, wenn durch das Vorhandensein des Polymers das Verhindern des Kontakts des wenigstens einen Typs Schadstoff mit der Haut > 70 % beträgt, der entsprechenden Fähigkeit ($\beta$) ein Wert von 0,7 auf der Skala von 0 bis 1 zugewiesen wird, und wenn herausgefunden wird, dass das Verhindern < 30 % beträgt, der entsprechenden Fähigkeit ($\beta$) ein Wert von 0,2 auf der Skala von 0 bis 1 zugewiesen wird, und wobei entsprechend die verbleibende Schädigung 0,3 und 0,8 beträgt.

7. Verfahren nach einem der vorhergehenden Ansprüche, wobei die Zusammensetzung wenigstens einen Inhaltsstoff umfasst, der dafür bekannt ist, wenigstens einen Typ Schadstoff, der auf der Haut vorhanden ist, zu entfernen.

8. Verfahren nach einem der vorhergehenden Ansprüche, wobei dann, wenn die Zusammensetzung ein Reinigungsmittel ist, das 75 bis 85 % des wenigstens einen Typs Schadstoff, der auf der Haut vorhanden ist, entfernt, der entsprechenden Fähigkeit ein Wert auf 0,4 auf der Skala von 0 bis 1 zugewiesen wird, und wenn die Zusammensetzung ein Reinigungsmittel ist, das 85 bis 95 % des wenigstens einen Typs Schadstoff, der auf der Haut vorhanden ist, entfernt, der entsprechenden Fähigkeit ein Wert von 0,7 auf der Skala von 0 bis 1 zugewiesen wird, und wenn die Zusammensetzung ein Reinigungsmittel ist, das 95 bis 100 % des wenigstens einen Typs Schadstoff, der auf der Haut vorhanden ist, entfernt, der entsprechenden Fähigkeit ein Wert von 0,8 auf der Skala von 0 bis 1 zugewiesen wird, und wobei entsprechend die verbleibende Schädigung 0,6, 0,3 und 0,2 beträgt.

9. Verfahren nach einem der vorhergehenden Ansprüche, wobei der PPF eine Zahl zwischen 0 und 75 ist.

**Revendications**

1. Procédé de détermination d'un facteur de protection contre la pollution (PPF) d'une composition cosmétique, comprenant :

(a) une étape de test, par toute méthode de test connue, de l'aptitude ($\alpha$) de la composition cosmétique à rendre inefficace au moins un type de polluant présent sur la peau et d'expression de celle-ci sur une échelle de 0 à 1, où (1 - $\alpha$) indique une détérioration résiduelle potentielle correspondante de la peau, dans laquelle ladite composition comprend au moins un ingrédient connu pour rendre inefficace ledit au moins un type de polluant et ledit au moins un ingrédient est un anti-inflammatoire ou antioxydant ;
(b) une étape de test, par toute méthode de test connue, de l'aptitude ($\beta$) de la composition cosmétique à inhiber le contact d'au moins un type de polluant avec la peau et d'expression de celle-ci également sur une échelle de 0 à 1, où (1 - $\beta$) indique une détérioration résiduelle potentielle correspondante de la peau dans laquelle ladite composition comprend au moins un ingrédient connu pour inhiber le contact d'au moins un type de polluant avec la peau et dans laquelle ledit ingrédient est un écran solaire, un promoteur de barrière ou un polymère, suivie de plus par ;
(c) une étape de test, par toute méthode de test connue, de l'aptitude ($\gamma$) de la composition cosmétique à éliminer au moins un type de polluant présent sur la peau, d'expression de celle-ci également sur une échelle de 0 à 1, où (1 - $\gamma$) indique une détérioration résiduelle potentielle correspondante de la peau, et de calcul du facteur de protection contre la pollution par l'équation F.

| F |
| --- |
| $PPF = -\dfrac{\log{(1-\alpha)} \times (1-\beta) \times (1-\gamma)}{0.047}$ |

2. Méthode selon la revendication 1, dans laquelle quand on trouve que ledit ingrédient présente un effet anti-inflammatoire comparativement plus faible ladite aptitude ($\alpha$) est de 0,2 et quand on trouve que ledit ingrédient présente un effet anti-inflammatoire comparativement plus fort ladite aptitude ($\alpha$) est de 0,75, et ladite détérioration résiduelle potentielle est proportionnellement de 0,8 à 0,25.

3. Méthode selon la revendication 1 ou 2, dans laquelle quand on trouve que ledit ingrédient présente un effet antioxydant comparativement plus faible ladite aptitude ($\alpha$) est de 0,2 et quand on trouve que l'ingrédient présente un effet anti-inflammatoire comparativement plus fort ladite aptitude ($\alpha$) est de 0,8, et la détérioration résiduelle potentielle est proportionnellement de 0,8 à 0,2.

4. Méthode selon l'une quelconque des revendications précédentes, dans laquelle si le SPF (facteur de protection contre le soleil) dudit écran solaire est supérieur à 15, l'aptitude ($\beta$) correspondante est de 0,5 et si le SPF dudit écran solaire est inférieur ou égal à 15, l'aptitude ($\beta$) correspondante est de 0,2 et la détérioration résiduelle potentielle est proportionnellement de 0,5 à 0,8.

5. Méthode selon l'une quelconque des revendications précédentes, dans laquelle quand un promoteur de barrière fort est présent l'aptitude ($\beta$) correspondante est de 0,7 et quand un promoteur de barrière faible est présent, l'aptitude (β) correspondante est de 0,2 et la détérioration résiduelle est proportionnellement dans l'intervalle de 0,3 à 0,8.

6. Méthode selon l'une quelconque des revendications précédentes, dans laquelle quand, en ce qui concerne la présence du polymère, on trouve que l'inhibition du contact dudit au moins un type de polluant avec la peau est >70 %, on attribue à l'aptitude (β) correspondante un score de 0,7 sur l'échelle de 0 à 1 et quand on trouve que ladite inhibition est <30 % on attribue à l'aptitude (β) correspondante un score de 0,2 sur l'échelle de 0 à 1 et la détérioration résiduelle est proportionnellement de 0,3 à 0,8.

7. Méthode selon l'une quelconque des revendications précédentes, dans laquelle ladite composition comprend au moins un ingrédient connu pour éliminer au moins un type de polluant présent sur la peau.

8. Méthode selon l'une quelconque des revendications précédentes, dans laquelle quand ladite composition est un nettoyant qui élimine de 75 à 85 % dudit au moins un type de polluant présent sur la peau on attribue à l'aptitude correspondante un score de 0,4 sur l'échelle de 0 à 1 et quand ladite composition est un nettoyant qui élimine de 85 à 95 % dudit au moins un type de polluant présent sur la peau on attribue à l'aptitude correspondante un score de 0,7 sur l'échelle de 0 à 1, et quand ladite composition est un nettoyant qui élimine de 95 à 100 % dudit au moins un type de polluant présent sur la peau on attribue à l'aptitude correspondante un score de 0,8 sur l'échelle de 0 à 1, et la détérioration résiduelle est proportionnellement de 0,6, 0,3 et 0,2.

9. Méthode selon l'une quelconque des revendications précédentes, dans laquelle ledit PPF est un nombre de 0 à 75.

## REFERENCES CITED IN THE DESCRIPTION

**Patent documents cited in the description**

- EP 1760440 A1
- KR 100373519 B
- US 2008187502 A1, Michelle Garay
- CN 08785201 A
- CN 106290176 A

**Non-patent literature cited in the description**

- **HERRLING, T.** The Radical Status Factor (RSF): a novel metric to characterize skin products. *Int J Cosmet Sci.,* August 2012, vol. 34 (4), 285-90